# EUROPÄISCHE PATENTSCHRIFT

(11) **EP 1 197 189 B1**
(45) Veröffentlichungstag und Bekanntmachung des Hinweises auf die Patenterteilung: **12.10.2005**
(21) Anmeldenummer: 01250351.2
(22) Anmeldetag: 08.10.2001
(51) Int. Cl.: A61F 2/06

(54) **Stent**
Stent
Stent

(30) Priorität: 10.10.2000 DE 10050971
(43) Veröffentlichungstag der Anmeldung: 17.04.2002
(73) Patentinhaber: BIOTRONIK Mess- und Therapiegeräte GmbH & Co Ingenieurbüro Berlin, 12359 Berlin (DE)
(72) Erfinder: Schaldach, Max, VERSTORBEN (DE); Lootz, Daniel, 18119 Warnemünde (DE); Koop, Karsten, 18055 Rostock (DE); Kranz, Curt, 14163 Berlin (DE)
(74) Vertreter: Eisenführ, Speiser & Partner

(56) Entgegenhaltungen:
- EP-A- 0 928 605
- WO-A-98/32412
- US-A- 5 928 280

## Beschreibung

Die vorliegende Erfindung betrifft einen Stent, insbesondere einen Koronarstent, zum Expandieren von einem ersten Zustand in einen aufgeweiteten zweiten Zustand, in dem er ein Gefäß aufgeweitet hält, mit einem rohrförmigen Körper, dessen Mantel von einer Anzahl in Längsrichtung des Stents verlaufender Stützabschnitte aus Stegelementen gebildet ist, die über Verbindungsstege verbunden sind. Dabei ist eine Anzahl von Stützabschnittgruppen mit wenigstens einem ersten Stützabschnitt und einem in Umfangsrichtung des Stents benachbarten zweiten Stützabschnitt vorgesehen, deren Stegelemente mäanderförmig in Längsrichtung des Stents verlaufen. Weiterhin greifen die ersten Angriffspunkte erster Verbindungsstege an dem ersten Stützabschnitt und die zweiten Angriffspunkte der ersten Verbindungsstege an dem zweiten Stützabschnitt an.

Bei einem Stent handelt es sich um ein so genanntes intraluminales Expansionselement, welches dazu eingesetzt wird, ein Gefäß, beispielsweise ein Blutgefäß, des menschlichen oder tierischen Körpers in einem aufgeweiteten Zustand zu halten. Hierzu wird der Stent in einem komprimierten ersten Zustand mittels eines entsprechenden Katheters an die aufgeweitet zu haltende Stelle im Gefäß herangeführt. Ist die Implantationsstelle erreicht, wird der Stent radial in einen aufgeweiteten zweiten Zustand expandiert. Bei so genannten ballonexpandierbaren Stents wird der Stent dabei mittels eines Ballonkatheters so stark aufgeweitet, dass er auf Grund plastischer Verformung auch nach Entfernen des Ballons seinen aufgeweiteten zweiten Zustand beibehält und somit das Gefäß abstützt. Bei den so genannten selbstexpandierenden Stents wird der Stent, beispielsweise durch einen Hüllkatheter, gegen eine Rückstellkraft in einem komprimierten ersten Zustand gehalten. Dieser Zwang wird an der Implantationsstelle gelöst, so dass der Stent von selbst seinen aufgeweiteten zweiten Zustand annimmt.

WO 98/32412 A beschreibt eine bistabile Federkonstruktion für einen Stent, bei dem ein eher starres Segment eine Deformation eines benachbarten, eher flexiblen Elements in eine erste Hauptrichtung verhindert. Hierdurch soll erreicht werden, dass sich die Struktur nur in eine senkrecht zur ersten Hauptrichtung stehende Richtung deformieren lässt. Weiterhin ist die Struktur derart beschaffen, dass sie eine erste mehr oder weniger stabile kollabierte Form und eine zweite mehr oder weniger stabile expandierte Form annehmen kann. Dadurch, dass sich die starren und flexiblen Elemente in Längsrichtung des Stents erstrecken können und die starren Segmente bei der Expansion nicht verformt werden, wird eine Längenkontraktion bei Expansion verhindert.

Als gattungsgemäßer Stent ist beispielsweise der Crown Palmaz-SchatzTM Kornnarstent der Fa. Cordis, Warren, NJ, US, bekannt, bei dem in Längsrichtung des Stents mäanderförmig verlaufende Stegelemente im Bereich von Wendepunkten der Stegelemente über in Umfangsrichtung des Stents verlaufende Verbindungsstege verbunden sind. Diese Stents sind zwar auf Grund ihrer Gestaltung mit den in Längsrichtung des Stents verlaufenden mäanderförmigen Stegelementen relativ flexibel, sie weisen jedoch den Nachteil auf, dass sie sich relativ stark bei Expansion vom ersten in den zweiten Zustand verkürzen. Dies ist unerwünscht, da sich hierbei zum einen ihre Lage im Blutgefäß ändert und zum anderen bei der Verkürzung unter Umständen erhebliche Belastungen auf das Blutgefäß ausgeübt werden können.

Der vorliegenden Erfindung greift auf die technische Lehre zurück, dass man einen gattungsgemäßen Stent mit zumindest reduzierter Verkürzung bei der Expansion erhält, wenn die ersten und zweiten Angriffspunkte der ersten Verbindungsstege in einer ersten Längsrichtung des Stents zueinander beabstandet sind und die ersten Verbindungsstege derart ausgebildet und angeordnet sind, dass sich der Abstand zwischen dem ersten und zweiten Angriffspunkt der ersten Verbindungsstege in der ersten Längsrichtung beim Expandieren des Stents zur Kompensation der Verkürzung der Stützabschnitte ändert. Mit anderen Worten bedeutet dies, dass die ersten Verbindungsstege derart ausgebildet und angeordnet sind, dass sich die Neigung der Verbindungslinie zwischen dem ersten und zweiten Angriffspunkt des ersten Verbindungssteges bezüglich der Umfangsrichtung des Stents beim Expandieren des Stents ändert.

Durch die Abstandsänderung zwischen den ersten Angriffspunkten der Verbindungsstege an dem ersten Stützabschnitt und den zweiten Angriffspunkten der Verbindungsstege am zweiten Stützabschnitt wird der erste Stützabschnitt bezüglich in Umfangsrichtung angrenzenden zweiten Stützabschnitts in Längsrichtung des Stents verschoben. Die Verschiebungen der Stützabschnitte in den Stützabschnittgruppen sind dabei so gewählt, dass sich über den Stentumfang eine mehr oder weniger starke Kompensation der Verkürzung der Stützabschnitte bei der Expansion des Stents ergibt. Der Grad der Kompensation ergibt sich dabei je nach dem Verhältnis dieser Verschiebungen in Längsrichtung zur Verkürzung der Stegelemente. Es versteht sich, dass hierbei auf Grund der Expansion des Stents eine mehr oder weniger starke Abstandsänderung der Angriffspunkte der Stegelemente in Umfangsrichtung des Stents erfolgt.

Der vorliegende Erfindung liegt daher die Aufgabe zu Grunde, einen gattungsgemäßen Stent zur Verfügung zu stellen, der die oben genannten Nachteile nicht oder zumindest in geringerem Maß aufweist und insbesondere zumindest eine reduzierte Verkürzung bei der Expansion aufweist.

Diese Aufgabe wird ausgehend von einem Stent gemäß dem Oberbegriff des Anspruchs 1 durch die im kennzeichnenden Teil des Anspruchs 1 angegebenen Merkmale gelöst.

Die mäanderförmig in Längsrichtung verlaufenden Stegelemente haben vorzugsweise einen periodischen Verlauf. Diese Verlauf kann beliebige Krümmungsverteilungen haben. So ist beispielsweise ein sinusförmiger Verlauf möglich. Die erfindungsgemäßen Stents zeichnen sich dadurch aus, dass wenigstens ein Stützabschnitt von einem in Längsrichtung des Stents mäanderförmig verlaufenden Stegelement gebildet ist, wobei je zwei in Längsrichtung des Stents benachbarte, zwischen den Wendepunkten verlaufende Stegelementabschnitte die Schenkel eines V bilden und die die V-förmig angeordneten Stegelementabschnitte vorzugsweise einen Winkel zwischen 80° und 100° einschließen Vorzugsweise liegt dieser Winkel bei etwa 90°. Dies bewirkt eine gleichmäßige Struktur des Stents mit guten Verformungseigenschaften sowohl in Längs- als auch in Umfangsrichtung. Diese Gestaltung bringt eine besonders gute Flexibilität des Stents bezüglich seiner Längsrichtung mit sich.

Die Stützabschnitte werden bevorzugt in Umfangsrichtung des Stents abwechselnd in der beschriebenen Weise zueinander verschoben. Es versteht sich jedoch auch, dass mehrere in Umfangsrichtung aufeinanderfolgende Stützabschnitte Blöcke ohne eine solche Kompensation bilden. Diese Stützabschnittblöcke sind dann in der erfindungsgemäßen Weise miteinander verbunden. Sie werden demgemäß blockweise zueinander verschoben, um wiederum insgesamt über den Stent eine mehr oder weniger starke Kompensation zu erzielen. Es versteht sich weiterhin, dass mehrere in Umfangsrichtung aufeinander folgende Stützabschnitte oder Stützabschnittblöcke in derselben Richtung verschoben werden können. Das ist dann lediglich erforderlich, dass in Umfangsrichtung weitere Stützabschnitte oder Stützabschnittblöcke vorgesehen sind, wie zueinander in der entgegengesetzten Richtung verschoben werden. Um eine Verdrillung bzw. Verzerrung zu vermeiden, wäre diese weiteren Stützabschnitte bzw. Stützabschnittblöcke dann um denselben Betrag in der entgegengesetzten Richtung zueinander verschoben.

Die Stützabschnitte werden wie erwähnt bevorzugt in Umfangsrichtung des Stents abwechselnd in der beschriebenen Weise zueinander verschoben. Hierzu verlaufen vorzugsweise wenigstens die Stegelemente eines ersten Stützabschnitts sowie der beiden in Umfangsrichtung zu beiden Seiten des ersten Stützabschnitts angeordneten zweiten Stützabschnitte mäanderförmig in Längsrichtung des Stents verlaufen und die ersten Angriffspunkte der ersten Verbindungsstege greifen an dem ersten Stützabschnitt und die zweiten Angriffspunkte der ersten Verbindungsstege an einem der zweiten Stützabschnitte an. Die ersten und zweiten Angriffspunkte der ersten Verbindungsstege sind in einer ersten Längsrichtung des Stents zueinander beabstandet sind und die ersten Verbindungsstege sind dabei derart ausgebildet und angeordnet, dass sich der Abstand zwischen dem ersten und zweiten Angriffspunkt der ersten Verbindungsstege in der ersten Längsrichtung beim Expandieren des Stents in der gleichen Weise ändert.

Durch die erfindungsgemäße gleichgerichtete Abstandsänderung zwischen den ersten Angriffspunkten der Verbindungsstege an dem ersten Stützabschnitt und den zweiten Angriffspunkten der Verbindungsstege an den beidseitig des ersten Stützabschnitts liegenden zweiten Stützabschnitten wird der erste Stützabschnitt bezüglich der zu beiden Seiten in Umfangsrichtung angrenzenden zweiten Stützabschnitten in Längsrichtung des Stents verschoben, wodurch sich je nach dem Verhältnis dieser Verschiebung in Längsrichtung zur Verkürzung des Stegelements eine mehr oder weniger starke Kompensation der Verkürzung für den gesamten Stent ergibt. Es versteht sich, dass hierbei auf Grund der Expansion des Stents eine mehr oder weniger starke Abstandsänderung der Angriffspunkte der Stegelemente in Umfangsrichtung des Stents erfolgt.

Die Abstandänderung zwischen den ersten Angriffspunkten der Verbindungsstege und den zugehörigen zweiten Angriffspunkten der Verbindungsstege soll in der gleichen Weise erfolgen, d. h. der Verlauf der Abstandänderung ist zu beiden Seiten des ersten Stützabschnitts gleich. Hierbei müssen allerdings die Beträge der Abstandänderung zu beiden Seiten nicht notwendigerweise dieselben sein. In diesem Fall sind die Verschiebungen in Längsrichtung zwischen den einzelnen Stegelementen über den gesamten Umfang vorzugsweise so gewählt, dass sich über den gesamten Stentumfang ein Ausgleich ergibt, um eine Verzerrung bzw. Verdrillung des Stents über seine Länge zu vermeiden. Vorzugsweise sind die Abstandänderungen jedoch zu beiden Seiten nicht nur hinsichtlich ihres zeitlichen Verlaufes, sondern auch hinsichtlich ihres Betrages identisch. Hierdurch wird eine gleichmäßige Verkürzungskompensation erreicht, ohne dass es dabei zu Verzerrungen bzw. Verdrillungen des Stents kommt.

In welcher Richtung die Abstandsänderung zwischen den Angriffspunkten der Verbindungsstege erfolgt oder ob gegebenenfalls auch eine Richtungsänderung bei der Abstandsänderung erfolgt, ist unerheblich. So kann sich der Abstand zwischen dem ersten und zweiten Angriffspunkt der ersten Verbindungsstege in der ersten Längsrichtung beim Expandieren des Stents insgesamt verringern oder vergrößern. Es ist lediglich erforderlich, dass am Ende der Expansion ein entsprechend große betragsmäßige Abstandsänderung vorliegt.

Bei bevorzugten Varianten des erfindungsgemäßen Stents sind die ersten Angriffspunkte und zusätzlich oder alternativ die zweiten Angriffspunkte der ersten Verbindungsstege im Bereich eines Wendepunkts, insbesondere am Wendepunkt, des betreffenden Stegelementes angeordnet, wodurch sich eine besonders einfache Gestaltung des Stents ergibt.

Die ersten Verbindungsstege können einen beliebigen Verlauf aufweisen. Vorzugsweise sind jedoch im wesentlichen geradlinig ausgebildet, da sich hiermit ein besonders einfach zu fertigender Stent ergibt, der zudem ein einfach nachvollziehbares und besonders gut reproduzierbares Verformungsverhalten aufweist.

Bevorzugte, weil einfach herzustellende Varianten des erfindungsgemäßen Stents zeichnen sich dadurch aus, dass die Verbindungslinie zwischen dem ersten und zweiten Angriffspunkt der ersten Verbindungsstege im wesentlichen in Längsrichtung des Stents verläuft.

Beigünstigen Weiterbildungen des erfindungsgemäßen Stents ist vorgesehen, dass die Stegelemente der ersten und zweiten Stützabschnitte im wesentlichen den selben periodischen Verlauf aufweisen und die Länge der ersten Verbindungsstege derart gewählt ist, dass die benachbarten Stegelemente im ersten Zustand des Stents um bis zu einer Viertelperiode zueinander in Längsrichtung des Stents versetzt sind. Hierdurch ergibt sich im expandierten Zustand eine besonders gleichmäßige und gute Verteilung der Stützstellen für das Gefäß.

Eine besonders vorteilhafte, weil gleichmäßige Verteilung der Schnittstellen für das Gefäß ergibt sich insbesondere dann, wenn die Stegelemente der ersten und zweiten Stützabschnitte im wesentlichen den selben periodischen Verlauf aufweisen und die Länge der ersten Verbindungsstege derart gewählt ist, dass die benachbarten Stegelemente im zweiten Zustand des Stents bezüglich der Längsrichtung des Stents zueinander im wesentlichen in Phase verlaufen.

Bei bevorzugten Varianten des erfindungsgemäßen Stents sind die Stegelemente zur Erhöhung der Flexibilität des Stents ausgebildet. Dies kann in vielfacher bekannter Weise erfolgen. Insbesondere kann dies vorzugsweise dadurch erzielt werden, dass wenigstens ein Stützabschnitt von einem Stegelement gebildet ist, dessen Krümmungsrichtung sich im Mittenbereich zwischen zwei Wendepunkten ändert, da hierdurch die Flexibilität der einzelnen Abschnitte des Stegelements erhöht wird.

Die vorliegende Erfindung betrifft weiterhin eine Anordnung aus einem Katheter zur Stentimplantation mit einem Stent nach einem der vorhergehenden Ansprüche. Hierbei kann es sich je nach Art des Stents um einen Ballonkatheter handeln, auf den der Stent aufgebracht, beispielsweise gecrimpt ist. Ebenso kann es sich um einen Hüllkatheter handeln, in dem ein als selbstexpandierender Stent ausgebildeter Stent in seinem ersten Zustand gehalten wird.

Weitere bevorzugte Ausgestaltungen der vorliegenden Erfindung ergeben sich aus den Unteransprüchen und der nachstehenden Beschreibung bevorzugter Varianten des erfindungsgemäßen Stents unter Bezugnahme auf die beigefügten Zeichnungen. Es zeigen:
- Figur 1: eine Draufsicht auf die Abwicklung des Mantels eine bevorzugten Ausführungsform des erfindungsgemäßen Stents;
- Figur 2: eine Draufsicht auf die Abwicklung des Mantels einer weiteren bevorzugten Ausführungsform des erfindungsgemäßen Stents.

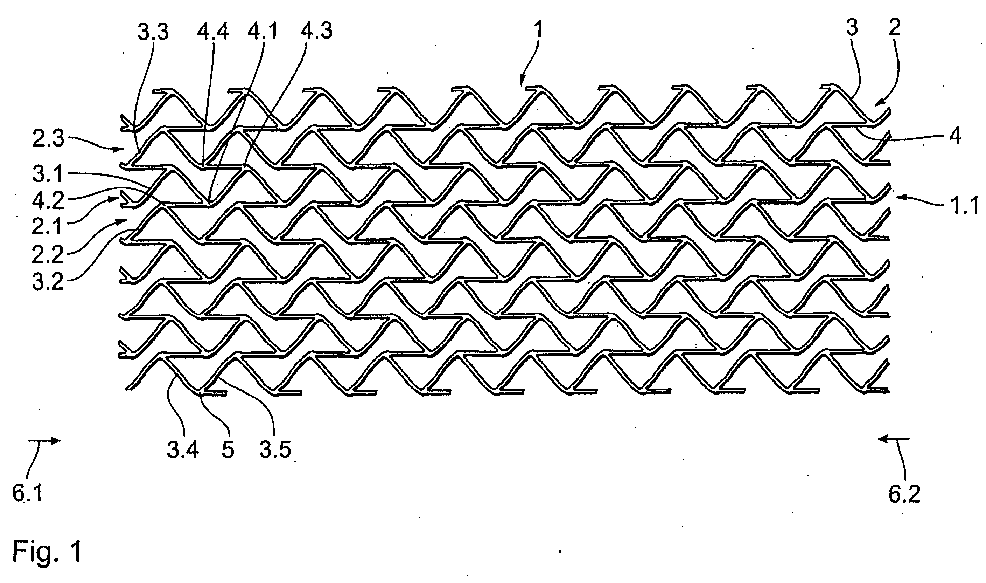

Figur 1 zeigt eine Draufsicht auf die Abwicklung des Mantels 1 einer bevorzugten Ausführungsformen des erfindungsgemäßen Stents mit Stützabschnitten 2, die von in Längsrichtung des Stents mäanderförmig verlaufenden Stegelementen 3 gebildet sind. Diese Stegelemente 3 sind in Umfangsrichtung des Stents über erste Verbindungsstege 4 miteinander verbunden.

Der Stent weist Stützabschnittgruppen 1.1 mit einem ersten Stützabschnitt 2.1 und einem hierzu in Umfangsrichtung benachbarten zweiten Stützabschnitt 2.2 auf, wobei an den ersten Stegelementen 3.1 der ersten Stützabschnitte 2.1 die ersten Angriffspunkte 4.1 und an den zweiten Stegelementen 3.2 der zweiten Stützabschnitte 2.2 die zweiten Angriffspunkte 4.2 der Verbindungsstege 4 angreifen. Die Verbindungsstege 4 greifen dabei jeweils an einem Wendepunkt 5 des Stegelements 3 an.

Die Verbindungsstege 4 verlaufen geradlinig in Längsrichtung des Stents, so dass die ersten und zweiten Angriffspunkte 4.1 und 4.2 der Verbindungsstege 4 in Längsrichtung des Stents zueinander beabstandet sind. Bei der Expansion des Stents in seinen ausgeweiteten zweiten Zustand ändert sich die Ausrichtung der Verbindungsstege 4 bezüglich der Umfangsrichtung des Stents. Die Neigung der Verbindungsstege 4 bezüglich der Umfangsrichtung des Stents verringert sich. Hiermit verringert sich auch der Abstand der ersten Angriffspunkte 4.1 und der zweiten Angriffspunkte 4.2 bezüglich der Längsrichtung des Stents, so dass das zweite Stegelement 3.2, d. h. der zweite Stützabschnitte 2.2, bezüglich des ersten Stegelements 3.1, d. h. bezüglich des ersten Stützabschnitts 2.1, in der ersten Längsrichtung 6.1 verschoben wird.

Diese Verschiebung zwischen dem ersten und zweiten Stützabschnitt 2.1 und 2.2 ist so groß, dass sie die Verkürzung der Stegelemente 3.1 und 3.2 bei der Expansion des Stents dahingehend kompensiert, dass der Abstand in Längsrichtung des Stents zwischen dem Endbereich des ersten Stegelements 3.1 in der ersten Längsrichtung 6.1 und dem Endbereich des zweiten Stegelements 3.2 in der entgegengesetzten zweiten Längsrichtung 6.2 im expandierten zweiten Zustand dem Abstand dieser Bereiche im nicht expandierten ersten Zustand entspricht.

Um eine Verdrillung bzw. Verzerrung des Stents bei der Expansion zu verhindern, grenzt an der anderen Seite des ersten Stegelements 3.1 ein weiteres zweites Stegelement 3.3 an, welches einen weiteren zweiten Stützabschnitt 2.3 bildet. Dieses ist über Verbindungsstege 4 mit dem ersten Stegelement 3.1 verbunden, wobei an dem ersten Stegelement 3.1 die ersten Angriffspunkte 4.3 und an dem zweiten Stegelement 3.3 die zweiten Angriffspunkte 4.4 angreifen. Die Verbindungsstege 4 sind dabei so angeordnet, dass die ersten Angriffspunkte 4.1 und 4.3 jeweils in der ersten Richtung 6.1 zu den zugehörigen zweiten Angriffspunkten 4.2 und 4.4 beabstandet sind. Die Verbindungsstege 4 weisen dieselbe Länge auf, so dass hierdurch erreicht wird, dass sich die zweiten Stegelemente 3.2 und 3.3 in Längsrichtung des Stents bei der Expansion nicht zueinander verlagern, da sich der Abstand zwischen den ersten Angriffspunkten 4.1, 4.3 und den zweiten Angriffspunkten 4.2 bzw. 4.4 der Verbindungsstege in derselben Weise ändert.

Die Stegelemente 3 und die Verbindungsstege 4 sind im gezeigten Beispiel so angeordnet, dass sich in Umfangsrichtung jeweils ein erstes und ein zweites Stegelement abwechseln. Hierdurch wird eine verzerrungsfreie Verkürzungskompensation erzielt. Es versteht sich jedoch, dass der anderen Varianten des erfindungsgemäßen Stents auch andere Konfigurationen vorgesehen sein können. So können beispielsweise statt eines ersten Stützabschnitts aus einem einzigen Stegelement Stützabschnitte aus mehreren in Umfangsrichtung aneinander anschließenden, nicht in der beschriebenen Weise verbundenen oder ausgestalteten Stegelementen vorgesehen sein. Der erste Stützabschnitte kann aber dann wiederum in der beschriebenen Weise über entsprechende Verbindungsstege mit zu beiden Seiten angrenzenden, gegebenenfalls entsprechend gestalteten zweiten Stützabschnitte verbunden sein, so dass insgesamt wieder eine entsprechende verzerrungsfreie Verkürzungskompensation erzielt wird.

Die Stegelemente 3 verlaufen in Längsrichtung des Stents periodisch, wobei je zwei in Längsrichtung des Stents benachbarte, zwischen den Wendepunkten 5 verlaufende Stegelementabschnitte 3.4 und 3.5 die Schenkel eines V bilden. Die Stegelementabschnitte 3.4 und 3.5 schließen dabei einen Winkel von 90° ein. Durch die gewählte Konfiguration wird eine besonders einfache Herstellbarkeit des Stents erzielt. Zudem ergibt sich hiermit eine besonders gute Flexibilität des Stents bezüglich seiner Längsachse.

Die Länge der Verbindungsstege 4 ist so gewählt, dass die benachbarten Stegelemente 3.1, 3.2 und 3.3 im expandierten Zustand des Stents bezüglich der Längsrichtung des Stents miteinander in Phase verlaufen. Hierzu ist die Länge der Verbindungsstege 4 ist so gewählt, dass die ersten und zweiten Stegelemente 3.1 und 3.2 bzw. 3.3 im dargestellten ersten Zustand des Stents um etwa ein Zehntel ihrer Periode zueinander in Längsrichtung verschoben angeordnet sind.

Die Stegelemente 3 sind zudem zur Erhöhung der Flexibilität des Stents ausgebildet, indem sich ihre Krümmungsrichtung im Mittenbereich zwischen zwei Wendepunkten 5 ändert. Diese leicht S-förmige Gestalt bewirkt eine geringere Steifigkeit der Stegelemente gegenüber in Längsrichtung des Stents wirkenden Kräften und damit eine erhöhte Flexibilität des Stents.

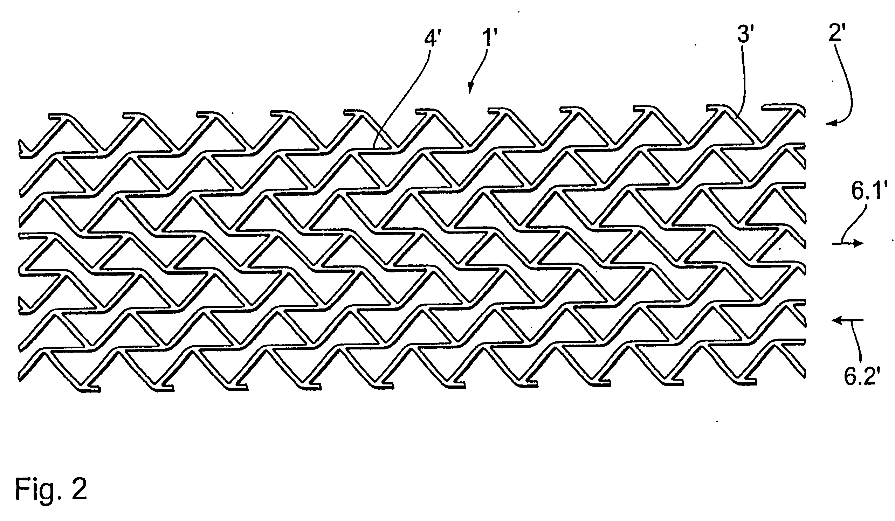

Figur 2 zeigt eine schematische Draufsicht auf die Abwicklung des Mantels 1' einer bevorzugten Ausführungsformen des erfindungsgemäßen Stents mit Stützabschnitten 2', die von in Längsrichtung des Stents mäanderförmig verlaufenden Stegelementen 3' gebildet sind. Diese Stegelemente 3' sind in Umfangsrichtung des Stents über erste Verbindungsstege 4' miteinander verbunden.

Diese Variante unterscheidet sich in ihren grundsätzlichen Aufbau und ihre grundsätzlichen Funktion nicht von der Variante aus Figur 1, so dass lediglich auf die Unterschiede eingegangen werden soll. Der Unterschied besteht darin, dass die Verbindungsstege 4' derart angeordnet sind, dass benachbarte Stegelemente 3' beim Expandieren des Stents paarweise jeweils in dieselbe erste Längsrichtung 6.1' verschoben werden, während die in Umfangsrichtung anschließenden nächsten beiden Stegelemente 3' beide jeweils in die entgegengesetzte zweite Längsrichtung 6.2 verschoben werden. Es sind dabei so viel Paare von Stegelementen 3' vorgesehen, dass sich insgesamt über den Umfang des Stents eine verdrillungsfreie Längenkompensation ergibt.

Es versteht sich, dass bei anderen Varianten auch eine andere Anzahl aneinander angrenzender jeweils in dieselbe Längsrichtung verschobener Stegelemente vorgesehen sein kann.

## Patentansprüche

1. Stent, insbesondere Koronarstent, zum Expandieren von einem ersten Zustand in einen aufgeweiteten zweiten Zustand, in dem er ein Gefäß aufgeweitet hält, mit einem rohrförmigen Körper, dessen Mantel (1; 1') von einer Anzahl in Längsrichtung des Stents verlaufender Stützabschnitte (2; 2') aus Stegelementen (3, 3.1, 3.2, 3.3; 3') gebildet ist, die über Verbindungsstege (4, 4') verbunden sind, wobei eine Anzahl von Stützabschnittgruppen (1.1) mit wenigstens einem ersten Stützabschnitt (2.1) und einem in Umfangsrichtung des Stents benachbarten zweiten Stützabschnitt (2.2, 2.3) vorgesehen ist, deren Stegelemente (3, 3.1, 3.2, 3.3; 3') mäanderförmig in Längsrichtung des Stents verlaufen, und wobei die ersten Angriffspunkte (4.1, 4.3) erster Verbindungsstege (4) an dem ersten Stützabschnitt (2.1) und die zweiten Angriffspunkte (4.2, 4.4) der ersten Verbindungsstege (4; 4') an dem zweiten Stützabschnitt (2.2, 2.3) angreifen, wobei die ersten und zweiten Angriffspunkte (4.1, 4.3, 4.2, 4.4) der ersten Verbindungsstege (4; 4') in Längsrichtung des Stents zueinander beabstandet sind und die ersten Verbindungsstege (4; 4') derart ausgebildet und angeordnet sind, dass sich der Abstand zwischen dem ersten und zweiten Angriffspunkt (4.1, 4.3, 4.2, 4.4) der ersten Verbindungsstege (4; 4') in Längsrichtung des Stents beim Expandieren des Stents ändert, **dadurch gekennzeichnet, dass** wenigstens ein Stützabschnitt (2, 2.1, 2.2, 2.3; 2') von einem in Längsrichtung des Stents mäanderförmig verlaufenden Stegelement (3, 3.1, 3.2, 3.3; 3') gebildet ist, wobei je zwei in Längsrichtung des Stents benachbarte, zwischen den Wendepunkten (5) verlaufende Stegelementabschnitte (3.4, 3.5) die Schenkel eines V bilden und die Stegelementabschnitte (3.4, 3.5) einen Winkel zwischen 80° und 100° einschließen.

2. Stent nach Anspruch 1, **dadurch gekennzeichnet, dass** wenigstens die Stegelemente (3.1) eines ersten Stützabschnitts (2.1) sowie der beiden in Umfangsrichtung zu beiden Seiten des ersten Stützabschnitts (2.1)angeordneten zweiten Stützabschnitte (2.2, 2.3) mäanderförmig in Längsrichtung des Stents verlaufen und die ersten Angriffspunkte (4.1, 4.3) erster Verbindungsstege (4) an dem ersten Stützabschnitt (2.1) und die zweiten Angriffspunkte (4.2, 4.4) der ersten Verbindungsstege (4) an einem der zweiten Stützabschnitte (2.2, 2.3) angreifen, wobei die ersten und zweiten Angriffspunkte (4.1, 4.3, 4.2, 4.4) der ersten Verbindungsstege (4) in einer ersten Längsrichtung (6.1) des Stents zueinander beabstandet sind und die ersten Verbindungsstege (4) derart ausgebildet und angeordnet sind, dass sich der Abstand zwischen dem ersten und zweiten Angriffspunkt (4.1, 4.3, 4.2, 4.4) der ersten Verbindungsstege (4) in Längsrichtung des Stents beim Expandieren des Stents in der gleichen Weise ändert.

3. Stent nach Anspruch 1 oder 2, **dadurch gekennzeichnet, dass** die ersten Angriffspunkte (4.1, 4.3) und/oder die zweiten Angriffspunkte (4.2, 4.4) der ersten Verbindungsstege (4) im Bereich eines Wendepunkts (5), insbesondere am Wendepunkt (5), des betreffenden Stegelementes (3, 3.1, 3.2, 3.3; 3') angeordnet sind.

4. Stent nach einem der vorhergehenden Ansprüche, **dadurch gekennzeichnet, dass** die ersten Verbindungsstege (4; 4') im wesentlichen geradlinig ausgebildet sind.

5. Stent nach einem der vorhergehenden Ansprüche, **dadurch gekennzeichnet, dass** die Verbindungslinie zwischen dem ersten und zweiten Angriffspunkt (4.1, 4.3, 4.2, 4.4) der ersten Verbindungsstege (4) im wesentlichen in Längsrichtung des Stents verläuft.

6. Stent nach Anspruch 1, **dadurch gekennzeichnet, dass** die Stegelementabschnitte (3.4, 3.5) einem Winkel von 90° enschließen.

7. Stent nach einem dervorhergehenden Ansprüche, **dadurch gekennzeichnet, dass** die Stegelemente (3.1, 3.2, 3.3; 3') der ersten und zweiten Stützabschnitte (2.1, 2.2, 2.3) im wesentlichen den selben periodischen Verlauf aufweisen und die Länge der ersten Verbindungsstege (4) derart gewählt ist, dass die benachbarten Stegelemente (3.1, 3.2, 3.3) im ersten Zustand des Stents um bis zu einer Viertelperiode zueinander in Längsrichtung des Stents versetzt sind.

8. Stent nach einem der vorhergehenden Ansprüche, **dadurch gekennzeichnet, dass** die Stegelemente (3.1,3.2, 3.3) der ersten und zweiten Stützabschnitte (2.1, 2.2, 2.3) im wesentlichen den selben periodischen Verlauf aufweisen und die Länge der ersten Verbindungsstege (4) derart gewählt ist, dass die benachbarten Stegelemente (3.1, 3.2, 3.3) im zweiten Zustand des Stents bezüglich der Längsrichtung des Stents zueinander im wesentlichen in Phase verlaufen.

9. Stent nach einem der vorhergehenden Ansprüche, **dadurch gekennzeichnet, dass** die Stegelemente (3, 3.1, 3.2, 3.3; 3') zur Erhöhung der Flexibilität des Stents ausgebildet sind.

10. Stent nach einem der vorhergehenden Ansprüche, **dadurch gekennzeichnet, dass** wenigstens ein Stützabschnitt (2, 2.1, 2.2, 2.3; 2') von einem Stegelement (3, 3.1, 3.2, 3.3; 3') gebildet ist, dessen Krümmungsrichtung sich im Mittenbereich zwischen zwei Wendepunkten (5) ändert.

11. Katheter zur Stentimplantation mit einem Stent nach einem der vorhergehenden Ansprüche.

## Claims

1. A stent, in particular a coronary stent, for expansion from a first condition into an expanded second condition in which it keeps a vessel expanded, comprising a tubular body, the circumferential surface (1; 1') of which is formed by a number of support portions (2; 2'), extending in the longitudinal direction of the stent and consisting of bar elements (3, 3.1, 3.2, 3.3; 3'), which are connected via connecting bars (4, 4'), wherein a number of support portion groups (1.1) is provided with at least one first support portion (2.1) and a second support portion (2,.2, 2.3) adjacent in the peripheral direction of the stent, the bar elements of which (3, 3.1, 3.2, 3.3; 3') extend in meandering manner in the longitudinal direction of the stent, and wherein the first points of application (4.1, 4.3) of first connecting bars (3) act on the first support portion (2.1) and the second points of application (4.2, 4.4) of the first connecting bars (3; 4') act on the second support portion (2.2, 2.3), wherein the first and second points of application (4.1, 4.3, 4.2, 4.4) of the first connecting bars (4; 4') are spaced from each other in the longitudinal direction of the stent and the first connecting bars (4; 4') are constructed and disposed in such a manner that the distance between the first and the second points of application (4.1, 4.3, 4.2, 4.4) of the first connecting bars (4;4') changes in the longitudinal direction of the stent during the expansion of the stent,
**characterised in that** at least one support portion (2, 2.1, 2.2, 2.3; 2') is formed by a bar element (3, 3.1, 3.2, 3.3; 3') that extends in meandering manner in the longitudinal direction of the stent, with two bar element portions (3.4, 3.5) adjacent in the longitudinal direction of the stent and extending between the turning points (5) forming the sides of a V, and the bar element portions (3.4, 3.5) enclosing an angle of between 80° and 100°.

2. A stent according to Claim 1,
**characterised in that** at least the bar elements (3.1) of a first support portion (2.1) and also of the two second support portions (2.2, 2.3) disposed in the peripheral direction on both sides of the first support portion (2.1) extend in meandering manner in the longitudinal direction of the stent and the first points of application (4.1, 4.3) of first connecting bars (4) act on the first support portion (2.1) and the second points of application (4.2, 4.4) of the first connecting bars (4) act on one of the second support portions (2.2, 2.3), with the first and second points of application (4.1, 4.3, 4.2, 4.4) of the first connecting bars (4) being spaced from each other in a first longitudinal direction (6.1) of the stent and the first connecting bars (4) being constructed and disposed in such a manner that the distance between the first and second points of application (4.1, 4.3, 4.2, 4.4) of the first connecting bars (4) in the longitudinal direction of the stent changing in the same manner during the expansion of the stent.

3. A stent according to Claim 1 or 2,
**characterised in that** the first points of application (4.1, 4.3) and/or the second points of application (4.2, 4.4) of the first connecting bars (4) are disposed in the vicinity of a turning point (5), in particular at the turning point (5) of the respective bar element (3, 3.1, 3.2, 3.3; 3').

4. A stent according to one of the preceding Claims,
**characterised in that** the first connecting bars (4; 4') have an substantially rectilinear construction.

5. A stent according to one of the preceding Claims,
**characterised in that** the connecting line between the first and the second points of application (4.1, 4.3, 4.2, 4.4) of the first connecting bars (4) runs substantially in the longitudinal direction of the stent.

6. A stent according to Claim 1,
**characterised in that** the bar element portions (3.4, 3.5) enclose an angle of 90°.

7. A stent according to one of the preceding Claims,
**characterised in that** the bar elements (3.1, 3.2, 3.3; 3') of the first and second support portions (2.1, 2.2, 2.3) have substantially the same periodic form and the length of the first connecting bars (4) is chosen so that the adjacent bar elements (3.1, 3.2, 3.3) in the first condition of the stent are shifted by a quarter period with respect to one another in the longitudinal direction of the stent.

8. A stent according to one of the preceding Claims,
**characterised in that** the bar elements (3.1, 3.2, 3.3) of the first and second support portions (2.1, 2.2, 2.3) have substantially the same periodic form and the length of the first connecting bars (4) is chosen so that the adjacent bar elements (3.1, 3.2, 3.3) run substantially in phase with each other in the second condition of the stent with respect to the longitudinal direction of the stent.

9. A stent according to one of the preceding Claims,
**characterised in that** the bar elements (3, 3.1, 3.2, 3.3; 3') are constructed to increase the flexibility of the stent.

10. A stent according to one of the preceding Claims,
**characterised in that** at least one support portion (2, 2.1, 2.2, 2.3; 2') is formed by a bar element (3, 3.1, 3.2, 3.3; 3'), the direction of curvature of which changes in the central region between two turning points (5).

11. A catheter for the implantation of the stent comprising a stent according to one of the preceding Claims.

## Revendications

1. Stent, en particulier un stent coronarien, expansible entre un premier état et un second état expansé, dans lequel il maintient un vaisseau sanguin à l'état dilaté, comportant un corps tubulaire, dont la paroi latérale (1 ; 1') est formée par une pluralité de tronçons de support (2 ; 2'), orientés dans le sens longitudinal du stent et constitués par des barrettes (3, 3.1, 3.2, 3.3, 3'), qui sont reliées entre elles par des barrettes de liaison (4, 4'), sachant qu'il est prévu une pluralité de groupes de tronçons de support (1.1), comportant au moins un premier tronçon de support (2.1) et un deuxième tronçon de support (2.2, 2.3) adjacent dans le sens périphérique du stent, dont les barrettes (3, 3.1, 3.2, 3.3 ; 3') s'étendent en forme de méandres dans le sens longitudinal du stent, et les premiers points de contact (4.1, 4.3) des premières barrettes de liaison (4) venant en prise sur le premier tronçon de support (2.1) et les deuxièmes points de contact (4.2, 4.4) des premières barrettes de liaison (4 ; 4') venant en prise sur le deuxième tronçon de support (2.2, 2.3), les premiers et les deuxièmes points de contact (4.1, 4.3, 4.2, 4.4) des premières barrettes de liaison (4 ; 4') étant écartés les uns des autres dans le sens longitudinal du stent, et les premières barrettes de liaison (4 ; 4') étant conçues et agencées de telle sorte que la distance entre le premier et le deuxième point de contact (4.1, 4.3, 4.2, 4.4) des premières barrettes de liaison (4 ; 4') varie dans le sens longitudinal du stent lors de l'expansion du stent, **caractérisé en ce qu'**au moins un tronçon de support (2, 2.1, 2.2, 2.3 ; 2') est formé par une barrette (3, 3.1, 3.2, 3.3 ; 3'), orientée en forme de méandres dans le sens longitudinal du stent, respectivement deux tronçons à barrettes (3.4, 3.5), adjacents dans le sens longitudinal du stent et constitués entre les points de retournement (5), constituant les branches d'un V et les tronçons à barrettes (3.4, 3.5) étant agencés en formant un angle entre 80° et 100°.

2. Stent selon la revendication 1, **caractérisé en ce qu'**au moins les barrettes (3.1) d'un premier tronçon de support (2.1), ainsi que les deux deuxièmes tronçons de support (2.2, 2.3), agencés dans le sens périphérique des deux côtés du premier tronçon de support (2.1), s'étendent en forme de méandres dans le sens longitudinal du stent et les premiers points de contact (4.1, 4.3) des premières barrettes de liaison (4) viennent en prise sur le premier tronçon de support (2.1) et les deuxièmes points de contact (4.2, 4.4) des premières barrettes de liaison (4) viennent en prise sur l'un des deuxièmes tronçons de support (2.2, 2.3), les premiers et les deuxièmes points de contact (4.1, 4.3, 4.2, 4.4) des premières barrettes de liaison (4) étant écartés les uns des autres dans une première direction longitudinale (6.1) du stent, et les premières barrettes de liaison (4) étant conçues et agencées de telle sorte que la distance entre le premier et le deuxième point de contact (4.1, 4.3, 4.2, 4.4) des premières barrettes de liaison (4) varie de la même manière dans le sens longitudinal du stent lors de l'expansion du stent.

3. Stent selon la revendication 1 ou 2, **caractérisé en ce que** les premiers points de contact (4.1, 4.3) et/ou les deuxièmes points de contact (4.2, 4.4) des premières barrettes de liaison (4) sont agencés dans la zone d'un point de retournement (5), en particulier au niveau du point de retournement (5) de la barrette (3, 3.1, 3.2, 3.3 ; 3') concernée.

4. Stent selon l'une quelconque des revendications précédentes, **caractérisé en ce que** les premières barrettes de liaison (4 ; 4') sont sensiblement rectilignes.

5. Stent selon l'une quelconque des revendications précédentes, **caractérisé en ce que** la ligne de liaison entre le premier et le deuxième point de contact (4.1, 4.3, 4.2, 4.4) des premières barrettes de liaison (4) est orientée sensiblement dans le sens longitudinal du stent.

6. Stent selon la revendication 1, **caractérisé en ce que** les tronçons à barrettes (3.4, 3.5) sont disposés en formant un angle de 90°.

7. Stent selon l'une quelconque des revendications précédentes, **caractérisé en ce que** les barrettes (3.1, 3.2, 3.3 ; 3') des premiers et deuxièmes tronçons de support (2.1, 2.2, 2.3) ont sensiblement le même tracé périodique et la longueur des premières barrettes de liaison (4) est choisie de telle sorte que, dans le premier état du stent, les barrettes (3.1, 3.2, 3.3) adjacentes sont déphasées entre elles jusqu'à un quart de période dans le sens longitudinal du stent.

8. Stent selon l'une quelconque des revendications précédentes, **caractérisé en ce que** les barrettes (3.1, 3.2, 3.3 ) des premiers et deuxièmes tronçons de support (2.1, 2.2, 2.3) ont sensiblement le même tracé périodique et la longueur des premières barrettes de liaison (4) est choisie de telle sorte que, dans le second état du stent, les barrettes (3.1, 3.2, 3.3) adjacentes sont sensiblement en phase entre elles par rapport au sens longitudinal du stent.

9. Stent selon l'une quelconque des revendications précédentes, **caractérisé en ce que** les barrettes (3.1, 3.2, 3.3 ; 3') sont réalisées pour augmenter la flexibilité du stent.

10. Stent selon l'une quelconque des revendications précédentes, **caractérisé en ce qu'**au moins un tronçon de support (2, 2.1, 2.2, 2.3 ; 2') est formé par une barrette (3, 3.1, 3.2, 3.3 ; 3') dont le sens de la courbure varie dans la zone centrale entre deux points de retournement (5).

11. Cathéter destiné à l'implantation d'un stent selon l'une quelconque des revendications précédentes.
